(19) Europäisches Patentamt / European Patent Office / Office européen des brevets

(11) **EP 3 693 900 A1**

(12) **EUROPEAN PATENT APPLICATION**

(43) Date of publication:
**12.08.2020 Bulletin 2020/33**

(51) Int Cl.:
**G06Q 10/04** *(2012.01)*    **G01C 21/34** *(2006.01)*

(21) Application number: **19156190.1**

(22) Date of filing: **08.02.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Nokia Technologies Oy**
**02610 Espoo (FI)**

(72) Inventors:
• **Luca Maria, Aiello**
  **Cambridge, Cambridgeshire CB30FA (GB)**
• **Daniele, Quercia**
  **Cambridge, Cambridgeshire CB30FA (GB)**

(74) Representative: **Swindell & Pearson Limited**
**48 Friar Gate**
**Derby DE1 1GY (GB)**

(54) **APPARATUS, METHODS AND COMPUTER PROGRAMS FOR DETERMINING A ROUTE**

(57)    The application relates to apparatus, methods and computer programs for determining routes for a user performing an activity. In examples of the disclosure there is provided an apparatus comprising means for detecting an input that causes selection of a category of activity wherein the category of activity defines an intended physiological outcome for a user performing the activity; and determining a route for the selected category of activity. The route comprises a plurality of segments and values of one or more parameters are assigned to one or more of the plurality of segments. The values of the parameters provide a respective level of association of the one or more parameters with the intended physiological outcome defined by the selected category of activity. Determining a route for the selected category of activity comprises selecting segments from the plurality of segments based on the values of the one or more parameters.

FIG. 4

**Description**

TECHNOLOGICAL FIELD

Examples of the disclosure relate to apparatus, methods and computer programs for determining routes. Some relate to apparatus, methods and computer programs for determining routes for a user performing an activity.

BACKGROUND

Users performing activities such as running or walking may wish to have a route planned for them. Different users may have different preferences for the types of route.

BRIEF SUMMARY

According to various, but not necessarily all, examples of the disclosure there may be provided an apparatus comprising means for: detecting an input that causes selection of a category of activity wherein the category of activity defines an intended physiological outcome for a user performing the activity; and determining a route for the selected category of activity; wherein the route comprises a plurality of segments and values of one or more parameters are assigned to one or more of the plurality of segments where the values of the parameters provide a respective level of association of the one or more parameters with the intended physiological outcome defined by the selected category of activity; and wherein determining a route for the selected category of activity comprises selecting segments from the plurality of segments based on the values of the one or more parameters.

Weights may be assigned to respective ones of the values, where the weights are dependent upon the selected category of activity and where respective weights provide an association between an expected physiological effect on the user traversing a respective one of the plurality of segments and the intended physiological outcome defined by the selected category of activity; and wherein determining a route for the selected category of activity may comprise selecting segments from the plurality of segments as a function of the weighted values of the one or more parameters.

The physiological outcome may comprise a mental condition of the user.

Selecting a category of activity may comprise selecting one of a predefined plurality of categories. The predefined categories may comprise any one or more of; mindfulness, vibrancy.

The different parameters may have different weights for the different categories of activities.

The parameters may comprise parameters which affect the user's physical or mental state as they are traversing the route.

The parameters may comprise any one or more of; smell, sound, scenery, ground, obstacles, traffic volume, safety.

At least one of the parameters may comprise a plurality of sub-components.

Determining a route for the selected category of activity may comprise selecting segments from the plurality of segments so as to provide a cumulative value of the one or more parameters for the selected category activity above a threshold level.

Determining a route for the selected category of activity may comprise selecting segments from the plurality of segments so as to optimise the cumulative values of the one or more parameters.

The cumulative value may comprise a weighted value.

The means may be configured to detect one or more inputs indicating whether or not a user considers the route to satisfy the selected category of activity.

The inputs may be provided in response to one or more queries provided to the user.

The one or more inputs indicating whether or not a user considers the route to satisfy the selected category of activity may be used to update the values of the parameters assigned to the plurality of segments.

The means may be configured to detect an input that causes selection of a route length.

The means may also be configured to detect an input that causes selection of a start point and an end point for the route.

The activity may comprise traversing a route over land.

The activity may comprise using at least some physical exertion.

The activity may comprise at least one of: running, walking, cycling.

According to various, but not necessarily all, examples of the disclosure there may be provided an apparatus comprising: processing circuitry; and memory circuitry including computer program code, the memory circuitry and the computer program code configured to, with the processing circuitry, cause the apparatus to: detect an input that causes selection of a category of activity wherein the category of activity defines an intended physiological outcome for a user performing the activity; and determine a route for the selected category of activity; wherein the route comprises a plurality of segments and values of one or more parameters are assigned to one or more of the plurality of segments where the values of the parameters provide a respective level of association of the one or more parameters with the intended physiological outcome defined by the selected category of activity; and wherein determining a route for the selected category of activity comprises selecting segments from the plurality of segments based on the values of the one or more parameters.

According to various, but not necessarily all, examples of the disclosure there may be provided a method comprising: detecting an input that causes selection of a category of activity wherein the category of activity defines an intended physiological outcome for a user performing the activity; and determining a route for the selected category of activity; wherein the route comprises a plurality of segments and values of one or more parameters are assigned to one or more of the plurality of segments where the values of the parameters provide a respective level of association of the one or more parameters with the intended physiological outcome defined by the selected category of activity; and wherein determining a route for the selected category of activity comprises selecting segments from the plurality of segments based on the values of the one or more parameters.

According to various, but not necessarily all, examples of the disclosure there may be provided a computer program comprising computer program instructions that, when executed by processing circuitry, cause: detecting an input that causes selection of a category of activity wherein the category of activity defines an intended physiological outcome for a user performing the activity; and determining a route for the selected category of activity; wherein the route comprises a plurality of segments and values of one or more parameters are assigned to one or more of the plurality of segments where the values of the parameters provide a respective level of association of the one or more parameters with the intended physiological outcome defined by the selected category of activity; and wherein determining a route for the selected category of activity comprises selecting segments from the plurality of segments based on the values of the one or more parameters.

A physical entity embodying the computer program as described above.

An electromagnetic carrier signal carrying the computer program as described above.

BRIEF DESCRIPTION

Some example embodiments will now be described with reference to the accompanying drawings in which:

Fig. 1    illustrates an example apparatus;
Fig. 2    illustrates an example electronic device;
Fig. 3    illustrates an example method; and
Fig. 4    illustrates an example method.

DETAILED DESCRIPTION

Examples of the disclosure relate to apparatus 101, methods and computer programs 113 for determining routes for a user performing an activity. In examples of the disclosure there is provided an apparatus 101 comprising means for detecting 301 an input that causes selection of a category of activity wherein the category of activity defines an intended physiological outcome for a user performing the activity; and determining 303 a route for the selected category of activity. The route comprises a plurality of segments and values of one or more parameters are assigned to one or more of the plurality of segments. The values of the parameters provide a respective level of association of the one or more parameters with the intended physiological outcome defined by the selected category of activity. Determining a route for the selected category of activity comprises selecting segments from the plurality of segments based on the values of the one or more parameters.

Examples of the disclosure therefore enable a route to be determined for a user performing an activity so as to enable a physiological outcome to be achieved. The physiological outcome could comprise a mental condition of the user. The mental condition could be a state such as mindfulness or vibrancy or any other suitable mental state.

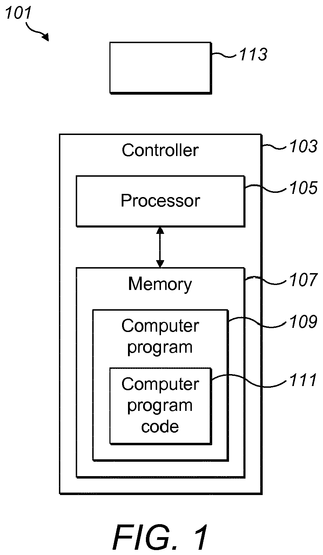

Fig.1 schematically illustrates an apparatus 101 according to examples of the disclosure. In the example of Fig. 1 the apparatus 101 comprises a controller 103. In the example of Fig. 1 the implementation of the controller 103 may be as controller circuitry. In some examples the controller 103 may be implemented in hardware alone, have certain aspects in software including firmware alone or can be a combination of hardware and software (including firmware).

As illustrated in Fig. 1 the controller 103 may be implemented using instructions that enable hardware functionality, for example, by using executable instructions of a computer program 109 in a general-purpose or special-purpose processor 105 that may be stored on a computer readable storage medium (disk, memory etc) to be executed by such a processor 105.

The processor 105 is configured to read from and write to the memory 107. The processor 105 may also comprise an output interface via which data and/or commands are output by the processor 105 and an input interface via which data and/or commands are input to the processor 105.

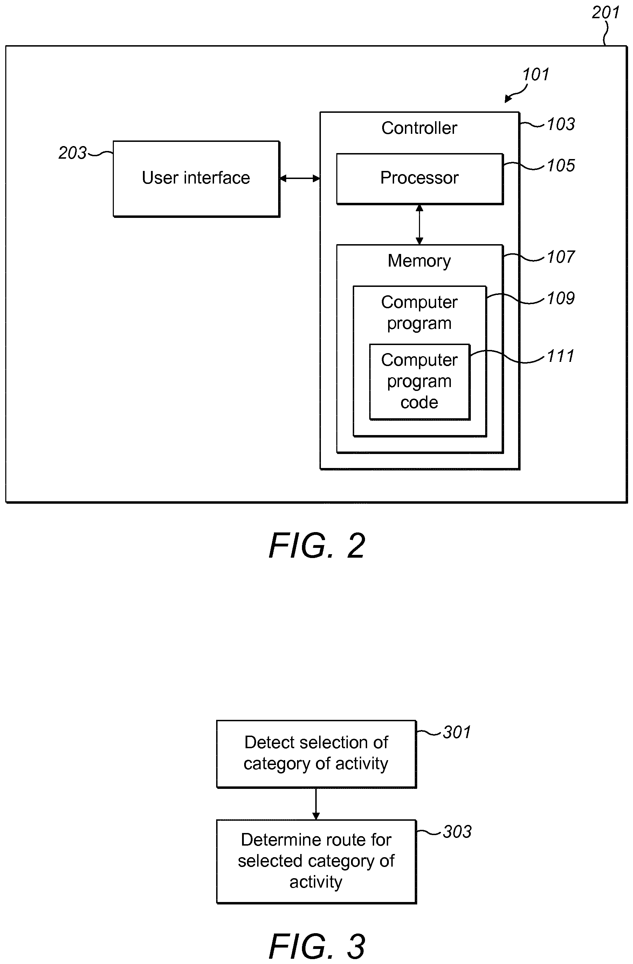

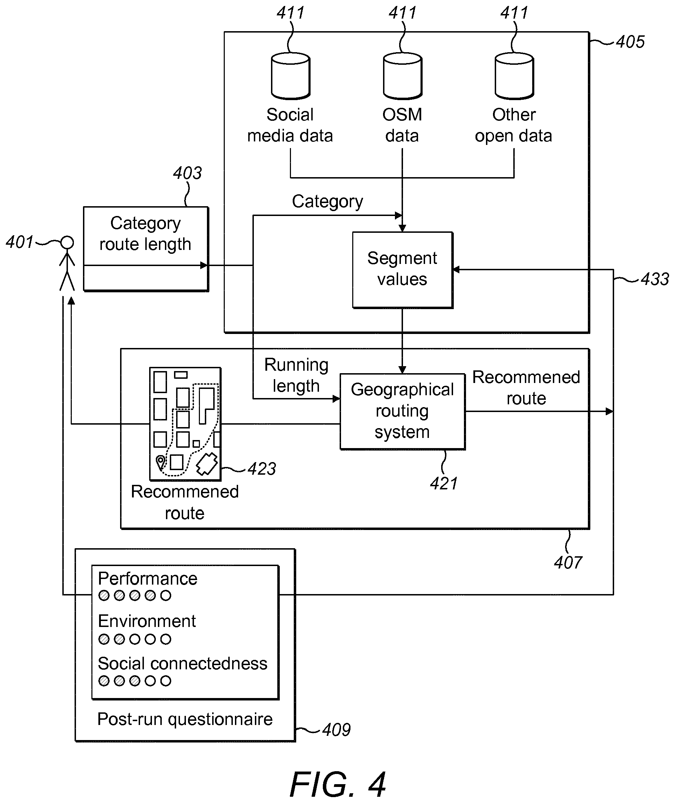

The memory 107 is configured to store a computer program 109 comprising computer program instructions (computer program code 111) that controls the operation of the apparatus 101 when loaded into the processor 105. The computer program instructions, of the computer program 109, provide the logic and routines that enables the apparatus 101 to perform the methods illustrated in Figs. 3 and 4. The processor 105 by reading the memory 107 is able to load and execute the computer program 109.

The apparatus 101 therefore comprises: at least one processor 105; and at least one memory 107 including computer program code 111, the at least one memory 107 and the computer program code 111 configured to, with the at least one processor 105, cause the apparatus 101 at least to perform: detecting 301 an input that causes selection of a category of activity wherein the category of activity defines an intended physiological outcome for a user performing the activity; and determining 303 a route for the selected category of activity; wherein the route comprises a plurality of segments and values of one or more parameters are assigned to one or more of the plurality of segments where the values of the parameters provide a respective level of association of the one or more parameters with the intended physiological outcome defined by the selected category of activity; and wherein determining a route for the selected category of activity comprises selecting segments from the plurality of segments based on the values of the one or more parameters.

As illustrated in Fig.1 the computer program 109 may arrive at the apparatus 101 via any suitable delivery mechanism 113. The delivery mechanism 113 may be, for example, a machine readable medium, a computer-readable medium, a non-transitory computer-readable storage medium, a computer program product, a memory device, a record medium such as a Compact Disc Read-Only Memory (CD-ROM) or a Digital Versatile Disc (DVD) or a solid state memory, an article of manufacture that comprises or tangibly embodies the computer program 109. The delivery mechanism may be a signal configured to reliably transfer the computer program 109. The apparatus 101 may propagate or transmit the computer program 109 as a computer data signal. In some examples the computer program 109 may be transmitted to the apparatus 101 using a wireless protocol such as Bluetooth, Bluetooth Low Energy, Bluetooth Smart, 6LoWPan (IP$_v$6 over low power personal area networks) ZigBee, ANT+, near field communication (NFC), Radio frequency identification, wireless local area network (wireless LAN) or any other suitable protocol.

The computer program 109 comprises computer program instructions for causing an apparatus 101 to perform at least the following: detecting 301 an input that causes selection of a category of activity wherein the category of activity defines an intended physiological outcome for a user performing the activity; and determining 303 a route for the selected category of activity; wherein the route comprises a plurality of segments and values of one or more parameters are assigned to one or more of the plurality of segments where the values of the parameters provide a respective level of association of the one or more parameters with the intended physiological outcome defined by the selected category of activity; and wherein determining a route for the selected category of activity comprises selecting segments from the plurality of segments based on the values of the one or more parameters.

The computer program instructions may be comprised in a computer program 109, a non-transitory computer readable medium, a computer program product, a machine readable medium. In some but not necessarily all examples, the computer program instructions may be distributed over more than one computer program 109.

Although the memory 107 is illustrated as a single component/circuitry it may be implemented as one or more separate components/circuitry some or all of which may be integrated/removable and/or may provide permanent/semi-permanent/ dynamic/cached storage.

Although the processor 105 is illustrated as a single component/circuitry it may be implemented as one or more separate components/circuitry some or all of which may be integrated/removable. The processor 105 may be a single core or multi-core processor.

References to "computer-readable storage medium", "computer program product", "tangibly embodied computer program" etc. or a "controller", "computer", "processor" etc. should be understood to encompass not only computers having different architectures such as single /multi- processor architectures and sequential (Von Neumann)/parallel architectures but also specialized circuits such as field-programmable gate arrays (FPGA), application specific circuits (ASIC), signal processing devices and other processing circuitry. References to computer program, instructions, code etc. should be understood to encompass software for a programmable processor or firmware such as, for example, the programmable content of a hardware device whether instructions for a processor, or configuration settings for a fixed-function device, gate array or programmable logic device etc.

As used in this application, the term "circuitry" may refer to one or more or all of the following:

(a) hardware-only circuitry implementations (such as implementations in only analog and/or digital circuitry) and
(b) combinations of hardware circuits and software, such as (as applicable):

(i) a combination of analog and/or digital hardware circuit(s) with software/firmware and
(ii) any portions of hardware processor(s) with software (including digital signal processor(s)), software, and memory(ies) that work together to cause an apparatus, such as a mobile phone or server, to perform various functions and

(c) hardware circuit(s) and or processor(s), such as a microprocessor(s) or a portion of a microprocessor(s), that requires software (e.g. firmware) for operation, but the software may not be present when it is not needed for operation.

This definition of circuitry applies to all uses of this term in this application, including in any claims. As a further example, as used in this application, the term circuitry also covers an implementation of merely a hardware circuit or processor and its (or their) accompanying software and/or firmware. The term circuitry also covers, for example and if applicable to the particular claim element, a baseband integrated circuit for a mobile device or a similar integrated circuit in a server, a cellular network device, or other computing or network device.

In some examples the apparatus 101 may be provided within an electronic device 201. An example electronic device 201 is shown schematically in Fig. 2. The example electronic device electronic device 201 comprises an apparatus 101 and a user interface 203. It is to be appreciated that only components of the electronic device 201 that are referred to in the following description are shown in Fig. 2 and that the electronic device 201 could comprise additional components in other examples of the disclosure. For instance the electronic device 203 could comprise one or more power sources, one or more transceivers or any other suitable components.

In examples of the disclosure the electronic device 201 could comprise a navigation device, a communication device or any other suitable processing device. In some examples the electronic device 201 could comprise a wearable electronic device or a portable electronic device which the user could take with them while they are performing their activity.

The apparatus 101 could be as shown in Fig. 1 and corresponding reference numbers are used for corresponding features.

The user interface 203 may comprise any means which enables a user to input information to the apparatus 101 and/or which enables information to be provided to the user from the apparatus 101. The user interface 203 could comprise a touch screen, a display, an audio output device, an audio input device or any other suitable means.

The user interface 203 could comprise means for enabling a user to make one or more inputs. For example the user interface 203 could comprise means for enabling a user to select a category of activity. The user interface 203 could also enable a user to indicate other criteria for the determined route such as the length, the start point, the end point and any other suitable criteria. In some examples the user interface 203 could also comprise means for enabling a user to provide feedback in relation to the determined route. For example, the user interface 203 could be configured to present one or more questions to the user relating to the determined route and the user interface 203 could be configured to enable a user to input answers to the one or more questions.

Fig. 3 illustrates an example method according to examples of the disclosure. The method could be implemented using apparatus 101 and electronic devices 201 as shown in Figs. 1 and 2 or by any other suitable means.

The method comprises, at block 301, detecting an input that causes selection of a category of activity. The input could be made via the user interface 203 or any other suitable means.

In some examples the input that causes selection of a category of activity could be a direct user input in which the user selects which category of activity they prefer. For instance, a user could be presented with a list of options and the user could select an option from this list. In some examples the input that causes selection of a category of activity could be an indirect user input in which information about the user's current physiological condition is obtained and a category of activity is recommended or selected based on the obtained information about the user's physiological condition. For instance one or more sensors could be configured to monitor physiological parameters and/or a user's behaviour which could then be used to determine a physiological state of the user such as stressed, tired, relaxed, upbeat or any other suitable condition. The category of activity could then be selected based on this determined physiological state.

The category of activity defines an intended physiological outcome for a user performing the activity. The physiological outcome may comprise a mental condition of the user. For example the physiological outcome could comprise a mental condition that the user wishes to achieve as a consequence of performing their chosen activity.

In some examples selecting a category of activity may comprise selecting one or more of a predefined plurality of categories of activity. In such examples the electronic device 201 may be configured to present a list of the available categories of activity to the user and the user could select one or more options from the list. In some examples the available categories of activity could comprise mindfulness, vibrancy or any other suitable physiological outcomes.

In some examples the method may also comprise detecting inputs that define other criteria of the route. For instance a user could use the user input to select a route length, a start point, an end point, time of day, intensity or difficulty for the activity or any other suitable criteria.

In some examples the user's current health could be taken into account when the route is being determined. For instance if a user is determined to have low fitness levels then the routes could be determined so as not to make the routes too difficult for the user. If the user is determined to have higher fitness levels then the routes could be determined so as to make them challenging for the user.

The activity that is to be performed could comprise traversing a route over land. The activity could comprise the user using at least some physical exertion. In some examples the activity could comprise at least one of running, walking, cycling or any other suitable activity.

At block 303 the method comprises determining a route for the selected category of activity. The route is determined so as to facilitate a user to achieve the selected physiological outcome when they traverse the route performing the activity.

In examples of the disclosure the route comprises a plurality of segments. In order to determine the route, values of one or more parameters are assigned to one or more of the plurality of segments. The values of the parameters may provide a respective level of association of the one or more parameters with the intended physiological outcome defined by the selected category of activity. In some examples the assigned values could provide an association between a physiological effect of the user traversing the segment and how much this physiological effect contributes to achieving the intended physiological outcome defined by the selected category. For instance the assigned values could provide an association between a physiological effect and the probability of the user achieving this physiological effect when they traverse this segment of the route.

In some examples weights may be assigned to respective ones of the values. The weights may be dependent upon the selected category of activity. The respective weights provide an association between an expected physiological effect on the user traversing the segment and the intended physiological outcome defined by the selected category of activity. The weighting could provide an association between a physiological effect and the probability of the user achieving this physiological effect when they traverse this segment of the route. The weights could be used to adjust values of parameters assigned to segments. The adjusting could be in accordance with the selected category of activity.

In some examples the weighting that is assigned to the values could be dependent upon the category of activity but independent of the segments. In such examples the same weighting factors could be applied to different segments for the same category of activity. These weighting factors could then be combined with the values of the parameters assigned to the segments to provide a numerical association between the segment and the physiological condition associated with the selected category of activity.

The parameters comprise any parameters which affect the user's physical or mental state as they are traversing the route. The parameters may comprise any parameters which may affect how likely the user is to achieve the intended physiological outcome.

In some examples the parameters could comprise physiological parameters. The physiological parameters could comprise parameters that can be perceived by the senses of the user such as noises, smells or scenery.

In some examples the values of the parameters could comprise data that is obtained from external sources. For instance a value of a safety parameter could be based on data relating to the type of road comprised within the segments, the availability of street lighting or any other information that could be obtained from an external information source.

In some examples the parameters could comprise any one or more of; smell, sound, scenery, ground, obstacles, traffic volume, safety or any other suitable parameter.

The values that are assigned to the these parameters for the available segments could be obtained from external information sources such as map applications, social media applications or any other suitable external information sources. In some examples the values that are assigned to these parameters for the available segments could be assigned, or adjusted, by feedback from one or more users.

In some examples the values of the parameters assigned to the segments may be dependent on the selected category of activity. In such examples the values may provide an indication of how important the parameter is to the selected category of activity. For instance a larger value may be assigned to parameters that are determined to be more important. The importance of a parameter may be associated with how likely it is that that parameter will help the selected physiological outcome to be obtained and/or how likely it is that parameter would prevent the physiological outcome from being obtained.

In some examples the weighting factors may be used to adjust the values of the parameters for the segments for the selected category of activity. In such examples the weighting factors could be used to provide an indication of how important the parameter is to the selected category of activity. For instance a larger weighting factor may be assigned to parameters that are determined to be more important. The weighting factors can therefore be used to adjust the values of the parameters depending upon the selected category of activity. In examples where weighting factors are used the value of the parameter assigned to a segment could be the same irrespective of the category of activity that is selected. The weighting factors are therefore used to adjust the values of the parameters to take the selected category of activity into account.

At least some of the different parameters will have different values and/or different weighting factors for the different categories of activities. For instance a segment that is considered to have natural aesthetics may have a high value and/or weighting factor for a mindful category but a low value and/or weighting factor for a vibrant category.

Conversely a segment that is considered to be busy and have a lot of people may have a high value and/or weighting factor for a vibrant category but a low value and/or weighting factor for a mindful category. The difference between the value and/or weighting factors for the different categories may depend upon factors such as the parameter, user indicated feedback and any other suitable information. The values of the parameters and/or the weighting factors assigned to the segments could also be determined by other criteria that the user has selected in relation to the route. For instance if a user selects a particular time of day for performing the activity this could determine the values and/or weighting factors of at least some of the parameters assigned to at least some of the segments. For instance, if the time of day is during daylight hours then segments with attractive scenery would be considered to contribute more to a mindful mental condition than they would when it is dark. The values of the parameters and/or weighting factors can be adjusted as needed to reflect these changes in conditions.

In some examples the values of the parameters assigned to the segments and/or the weighting factors could also be determined by the type of activity that the user is performing. For instance a segment could be considered to have high levels of safety for a runner but have a low level of safety for a cyclist.

In some examples one or more of the parameters could comprise one or more sub-components. In such examples different values could be provided for the one or more sub-components. The values of the different sub-components could then be combined to obtain a total value for the parameter. For example a category of smell could be divided into sub-components of different types of smells. The total value for the parameter of smell could then be obtained by averaging the values of the sub-components, by obtaining a weighted average of the values of the sub-components or by any other suitable method.

The values that are assigned to the different parameters could comprise positive or negative values. For instance a positive value could be used where the parameter is expected to help to achieve the intended physiological outcome and negative values could be used where it is considered that the parameters would not be expected to help, or would be expected to hinder, the user from achieving the intended physiological outcome.

In order to determine a route for the selected category of activity one or more segments are selected from the plurality of segments so as to produce a route of the desired length and having the desired start and end points. In examples of the disclosure the segments are selected based on the values of the one or more parameters. In some examples the segments may be selected based on the weighted values of the one or more parameters. As the values or weighted values are determined by the selected category of activity the segments that are selected are therefore also dependent upon the selected category of activity. The selection of different categories of activities would therefore cause different routes to be determined.

In examples of the disclosure the route may be determined by selecting appropriate segments from the available segments. Any suitable algorithm or process may be used to determine the route. In some examples the route may be determined by selecting segments from the plurality of segments so as to provide a cumulative value of the one or more parameters for the selected category above a threshold level. The cumulative value could comprise a weighted value. In such examples a route could be considered to satisfy the criteria of the selected category of activity if the total values of the segments within the route are above a threshold level. A user could then be provided with one or more options of routes that satisfy their selected category of activity.

In some examples determining a route for the selected category of activity may comprise selecting segments from the plurality of segments so as to optimise the cumulative values of the one or more parameters for the selected category. The cumulative value could comprise a weighted value. In such examples the route with the optimal cumulative value for the parameters could be calculated and then presented to the user.

It is to be appreciated that the method could also comprise additional blocks that are not shown in Fig. 3. For instance, in some examples the method could comprise updating the values and/or weightings of the parameters assigned to the plurality of segments. For instance a user could use the user interface 203 to make one or more inputs indicating whether or not they consider the route to satisfy the selected category. For example, the user could make the user inputs after they have completed the route and could then provide an indication as to how well the intended physiological outcome has been obtained. In some examples the user inputs could be provided in response to one or more queries provided to the user. These inputs could then be used to update one or more of the values of the parameters assigned to the plurality of segments and/or to update the weightings applied to the values of the parameters.

Fig. 4 illustrates another example method that could be implemented using apparatus 101 and electronic devices 201 as shown in Figs. 1 and 2 or any other suitable apparatus 101 and electronic devices 201. In the example of Fig. 4 the user 401 is looking for a route to go on a run. It is to be appreciated that in other examples the user 401 might wish to engage in a different type of activity such as walking or cycling.

In the implementation of Fig. 4 at block 403, the user 401 makes a user input to select a category of activity. The user 401 could select a category of activity from a list of available categories of activity.

The categories that are available for the user 401 to select may be determined by the motivations of different types of users 401. For instance, a first type of user 401 might prefer mindful routes. A mindful route could be a route that comprises natural settings and beautiful sceneries and other sensory perceptions that are pleasing or relaxing for the user 401. This could help the user 401 to obtain a calm and relaxed mental condition. A second type of user 401 might prefer vibrant routes. A vibrant route could be a route that incorporates urban settings and is likely to incorporate other people. The vibrant route could comprise steady ground. This could help the user 401 to obtain an energized mental condition.

In the example of Fig. 4 the user 401 can select whether they wish to have a vibrant run or a mindful run. It is to be appreciated that other types of category of activity could be available for selection by the user 401 in other examples of the disclosure.

At block 403 the user 401 could also make user inputs to define other criteria of the route. For instance the user 401 could define the route length. In some examples the user 401 could also define a start and end point for the route. In some examples the start point could be the same as the end point so that the route defines a closed circuit. In other examples the start point could be different to the end point, for instance the user 401 could be looking for a route home from work that helps them achieve the mental condition defined by the selected category of activity.

Other criteria that could be selected by the user 401 could be the type of activity that the user 401 is performing. For instance, the user 401 could indicate whether they will be running, walking or cycling or performing any other suitable activity. In some examples the user 401 could indicate the time that they wish to perform the route. In some examples the user 401 could indicate that they wish to perform the route immediately or in some cases they may be planning ahead and wish to perform the route later that day or on another day.

At block 405 values of one or more parameters are assigned to one or more of the plurality of the segments that are available to form part of the route for the user 401. The values of the parameters that are assigned to the segments of a route provide a respective level of association of the one or more parameters with the intended physiological outcome defined by the selected category of activity.

In the example of Fig. 4 the values of the parameters are determined by the category of activity. In this example the same parameter can be assigned different values depending upon the category of activity that has been selected. In some examples other criteria that the user 401 has selected could also influence the values assigned to the parameters. For instance, criteria such as the time of day and type of activity could also affect the values of the one or more parameters.

In the example of Fig. 4 the values of the parameters are assigned to a segment by determining a measured value and then combining the measured value with one or more appropriate weighting factors.

The measured values for the one or more parameters could be determined from one or more external information sources 411. The external information sources could comprise social media data, map applications such as open street map or any other type of data that can be accessed by the apparatus 101.

In some examples the measured values of the one or more parameters could also be determined and/or adjusted from feedback or other information from the user 401. This feedback could be stored in the apparatus 101 or could have been shared using social media or any other suitable sources.

The measured values for the parameters could be independent of the category of activity. For instance a safety level could be measured and could be the same for all categories of activities. The measured value could then be adjusted using a weighting factor to take into account how important this parameter is for the selected category of activity.

In the example of Fig. 4 a weighting may also be assigned to one or more respective values of the parameters. The weights may be dependent upon the selected category of activity. Respective weights may provide an association between an expected physiological effect on the user traversing the segment and the intended physiological outcome defined by the selected category of activity. In some examples the weighting could provide an indication of how important the parameter is considered to be for the selected category of activity. This weighting could be determined from information obtained from surveys, from social media, from online mapping applications or from any other suitable information source. The weighting could be used to adjust the measured value of a parameter in accordance with a selected category of activity.

In the example shown in Fig. 4 seven different parameters are used. The different parameters define different properties of the available segments that affect the physiological state of a user 401. In the example of Fig. 4 the different parameters comprise smell, sound, scenery, ground, obstacles, traffic, and safety. The values that are assigned to each of the parameters are different for the different categories of activity. For instance, beautiful scenery is considered to be more important for a mindful route than for a vibrant route and so a segment which is considered to have beautiful scenery would have a higher weighting for the scenery parameter when the user 401 requests a mindful route than when the user 401 requests a vibrant route.

Table 1 shows example weighting factors for the seven different parameters for the segments that could be used in some examples of the disclosure.

Table 1

| Parameter | Sub-component | $\beta_M$ | $B_V$ | Proxy |
|---|---|---|---|---|
| Smell | Nature | 1.80 | 1.40 | $f_{nature}^{smell}$ |
| $\alpha_M$=1.50 | Food | -0.64 | -0.17 | $f_{food}^{smell}$ |
| $\alpha_V$=0.40 | Emissions | -1.80 | -1.40 | $f_{emissions}^{smell}$ |
| | Chemical | -1.80 | -1.40 | $f_{chemical}^{smell}$ |
| | Synthetic | -1.30 | -0.81 | $f_{synthetic}^{smell}$ |
| | Animals | -0.23 | -0.19 | $f_{animals}^{smell}$ |
| | Odourless | 1.40 | 0.89 | - |
| Sound | Natural | 1.70 | 1.10 | $f_{food}^{sound}$ |
| $\alpha_M$=1.20 | People | 0.08 | 0.10 | $f_{people}^{sound}$ |
| $\alpha_V$=-0.06 | Transport | -1.30 | -0.68 | $f_{transport}^{sound}$ |
| | Music | 0.81 | 0.67 | $f_{music}^{sound}$ |
| | Quiet | 1.40 | 0.89 | $f_{quiet}^{sound}$ |
| Scenery | Natural | 1.90 | 1.50 | beauty |
| $\alpha_M$=1.50 | River | 1.80 | 1.40 | |
| $\alpha_V$=0.42 | Urban | 0.04 | 0.55 | |
| | Beach | 0.76 | 0.61 | |
| | Industrial | -1.10 | -0.43 | |
| Ground | Grass | 0.58 | 0.22 | $OSM_{grass}^{ground}$ |
| $\alpha_M$=1.60 | Pavement | 0.92 | 1.10 | $OSM_{pavement}^{ground}$ |
| $\alpha_V$=0.74 | Sand | -0.37 | -0.39 | $OSM_{sand}^{ground}$ |
| | Park | 1.60 | 1.30 | $OSM_{park}^{ground}$ |
| Obstacles $\alpha_M$=1.60 $\alpha_V$=0.39 | Absence of obstacles | - | - | $OSM_{obstacles}$ |
| Traffic $\alpha_M$=1.70 $\alpha_V$=0.48 | Absence of traffic | - | - | $OSM_{way\_type}$ |

(continued)

| Parameter | Sub-component | $\beta_M$ | $B_V$ | Proxy |
|---|---|---|---|---|
| Safety<br>$\alpha_M$=1.70<br>$\alpha_V$=0.58 | Safety | - | - | *Safety* |

This table lists the seven different parameters that are used in the example of Fig. 4. Each of these parameters has a first weighting factor $\alpha$. The weighting factor $\alpha$ provides an association between the parameters and the intended physiological outcome defined by the selected category of activity. In this example the larger the first weighting factor $\alpha$ the more significant the association between the parameters and the intended physiological outcome defined by the selected category of activity is. That is, a large first weighting factor $\alpha$ indicates that the parameter is likely to help a user 401 achieve the intended physiological outcome while a small first weighting factor $\alpha$ indicates that the parameter is unlikely to help a user 401 achieve the intended physiological outcome.

In the example of table 1 some of the first weighting factors $\alpha$ have negative values. This could provide an indication that the parameters do not help or do not contribute to a user achieving the intended physiological outcome defined by the selected category of activity.

In this example the first weighting factors $\alpha$ of the parameters are different for the different available categories of activities. This means that the first weighting factors $\alpha$ are dependent upon on the category of activity that has been selected.

In the example shown in table 1 at least some of the parameters have a plurality of sub-components. For instance the parameter of smell has seven sub-components of nature, food, emissions, chemical, synthetic, animals and odourless and the parameter of sound has five sub-components of natural, people, transport, music and quiet.

Each of the parameters or sub-components of the parameters is assigned a sub-weighting factor $\beta$. The sub-weighting factors $\beta$ are different for the different categories. The sub-weighting factor $\beta$ can have a positive value or a negative value depending on whether or not it will contribute to achieving the intended physiological condition defined by the selected category of activity.

In some examples the sub-weighting factor $\beta$ for the different sub-components may be combined with the first weighting factor $\alpha$ and a measured value for a segment to provide an overall value for the parameter for the segment. The sub-weighting factors $\beta$ could be combined linearly or using any other suitable process.

The proxy column in table 1 lists the names of the variables that are used to capture the respective parameters and their sub-components.

In the example of Fig. 4 the values of the parameters are used to determine a value for the available segments. In some examples the value for a segment may be determined by combining the assigned values for the different parameters.

In some examples of the disclosure the overall value for a segment j may be given by equation (1)

$$value(j) = \frac{1}{\sum_i^N (\alpha_{M|V}^i \cdot parameter_i(j))} \tag{1}$$

Where $\alpha$ represents the first weighting factor for the parameter for the selected category, and *parameter$_i$ (j)* represents a combination of the measured values of the sub-components of the parameters and N represent the total number of parameters.

In examples where the parameter has no sub-components then the overall value could be given by a combination of the first weighting factor $\alpha$ that is assigned to the parameter for that category and the measured value for that segment.

In examples where the parameter has a plurality of sub-components then *parameter$_i$ (j)*, the combination of the values of the sub-components of the parameter may be a combination of the weighting factors assigned to each of the sub-components and the measured parameters for each of the segments. In such examples *parameter$_i$ (j)* could be given by equation (2)

$$parameter_i(j) = \frac{1}{N} \cdot \sum_i^N (\beta_{M|V}^i \cdot sub-component_i(j)) \tag{2}$$

Where N is the number of sub-components and $\beta$ is the weighting factor assigned to the sub-component and *sub-component$_i$(j)* is the measured value for the sub-component.

In some examples the values for the parameters could be normalised so as to make the different parameters comparable. In some examples the values for the parameter could be normalised within the range [0, 1] using min-max optimisation or any other suitable normalisation process.

The overall values obtained using these weighting factors and measured parameter values therefore give values that can be assigned to the segments. These values can then be used to enable a route to be determined.

The measured values of the parameters can be obtained using any suitable information sources 411. For instance, if a user is looking to plan a route in a densely populate area such as a city or any other suitable populated area, then there may be sufficient information available via social media data and other open data to enable the values of the parameters to be determined.

To obtain the measured values for the smell parameter and the sound parameter textual tags attached to geo-referenced social media pictures were mined to obtain an estimation of the relative intensity of different types of smells and sounds that are likely to be perceived on a given segment. The measured value for each smell sub-component c in table 1 could be calculated using equation (3)

$$f_c^{smell}(j) = \frac{\#tags\ in\ segment\ j\ of\ smell\ sub-component\ c}{\#tags\ in\ segment\ j\ of\ any\ smell\ sub-component} \qquad (3)$$

A similar technique and equation can be used to determine the values for the sound sub-components.

The values for the scenery parameter can also obtained from the same geo-referenced social media pictures that are used to obtain the values for the smell parameter and the sound parameter. The geo-referenced social media pictures were used to estimate the aesthetic appeal of a segment. The aesthetic appeal of a street could be determined from factors such as the frequency with which the segment has been photographed, the proportion of the textual tags using positive words and the proportion of the textual tags using negative words. For instance, if a segment is photographed with a high frequency then it is likely that this segment is visually interesting.

The measured value for each beauty of each scenery sub-component for a segment j could be calculated using equation (4)

$$beauty(j) = 0.03 \cdot \log(\#pics\ in\ j) + 0.20 \cdot f_p(j) - 0.21 \cdot f_n(j) \qquad (4)$$

Where *#pics in j* is the number of pictures that have been taken in the segment j, $f_p(j)$ is the fraction of textual tags that contain positive emotion words and $f_n(j)$ is the fraction of textual tags that contain negative emotion words.

To obtain the measured values for the parameters such as ground, obstacles and traffic it is noted that these properties are strongly linked to the types of street found on a segment. Map applications such as Open Street Map (OSM) can therefore be used to obtain information about the street types found in the respective segments. This type of map application can also be used to obtain similar information about other cities and locations around the world.

To obtain the measured values for the obstacle parameter in table 1 the inverse count for traffic signals, stop signals, and give way signals can be computed. These obstacles could be labelled in the segments within the map application data. The labels may be provided as annotations, metadata or any other suitable type of data.

To obtain values for the traffic parameter in table 1 information relating to the type of street or roads comprised within a segment were obtained from map application data. The type of road could be a motorway, dual carriageway, main road, side road, country lane or any other type of road. The type of road within a segment can then be used to estimate the average traffic flow in that road.

To obtain the measured values for the parameters shown in table 1 the types of road or street can be grouped into three different sets reflecting different traffic flows. In this example the different traffic flows were low traffic; high traffic, and extreme traffic. In addition to this grouping the labels on the map data were analysed to identify roads that have sidewalks. The segments can then be assigned traffic parameter values dependent on which set of traffic flow the roads on the segment are grouped into. The measured values may be highest for low traffic roads or streets and lowest for high traffic roads or streets. Furthermore, streets with extreme traffic and those with high traffic and no sidewalk were removed and never considered by the routing algorithm.

To obtain measured values for the safety parameter publically available official crime data can be used. For instance, the application programming interface (API) of the crime and policing portal of England was used to retrieve data relating to the crimes reported within a 200m area of a segment in a given year or other suitable time period. The value for the safety parameter can then computed as the inverse of the number of crimes that were reported to have occurred within the 200m area of the segment. In this example crimes against a person, such as violence and robbery, can be counted. Other types of incident could be counted in other examples of the disclosure. It is also to be appreciated that other ranges could be used for the area around the segment.

It is to be appreciated that variations of these methods and/or other methods for obtaining measured values of parameters could be used in other examples of the disclosure. The measured values of the parameters assigned to the segments may be stored in the memory 107 of the apparatus 101. In some examples the measured values of the parameters assigned to the segments may be stored in one or more external devices which may be accessed by the apparatus 101 as needed.

At block 407 a geographical routing system 421 is used to provide a recommended route for the user 401. The geographical routing system 421 could be implemented using computer program code 111 within the apparatus 101 or by any other suitable means. In some examples the geographical routing system 421 could be implemented one or more external devices which may be accessed by the apparatus 101 as needed.

The geographical routing system 421 uses the values assigned to the parameters for the different segments to determine a recommended route. The geographical routing system 421 uses the values corresponding to the selected category of activity to determine the recommended route.

The geographical routing system 421 can receive data indicative of the available segments and the corresponding parameter values as an input. The geographical routing system 421 can also receive other information as an input. For instance the geographical routing system 421 can receive information indicating the start and end point of the route and the desired route length. This information could be input by the user 401 at block 403 or could be obtained at any other suitable point.

The route that is recommended may be determined by selecting segments from the plurality of available segments based on the one or more parameters assigned to the segments. In some examples the values can be weighted based on the length of the segments and/or the expected time it would take the user 401 to travel through the segment.

The geographical routing system 421 could determine the route by selecting segments so as to optimize the cumulative value for the parameters on the route. In some examples the optimal value could be a maximum value. In some examples the geographical routing system 421 could determine the route by selecting one or more routes that have a cumulative value for the parameters that is above a predetermined threshold. The cumulative values could be a weighted value that is obtained by applying weightings to one or more of the measured parameter values. This enables different routes to be recommended to the user 401 depending on the category of activity that the user 401 has selected.

The recommended route may be provided to the user in any appropriate manner. In some examples an image 423 of the recommended route could be presented on a display.

At block 409 the apparatus 101 is configured to enable a user 401 to give feedback on the recommended route. In the example of Fig. 4 the feedback is given by enabling the user 401 to answer a series of questions. The questions may be provided via a user interface 203. Other means for enabling the user 401 to provide feedback could be used in other examples of the disclosure.

In the example of Fig. 4 the user 401 is presented with three questions. These are three questions that have been determined to capture all of the factors that influence the user's perception of the route. The first question relates to performance & achievement, which is concerned with achievement of goals relating to the activity such as pace or distance and the confidence that the user 401 has in being able to attain those goals. The second question relates to the perceived environment which is concerned with the user's perceptions of the environment of the route such as the scenery and the cleanliness. The third question relates to social connectedness which relates to how a user 401 feels in relation to other people when following the route.

In the example of Fig. 4, at block 409, the user interface 203 is configured to present the user 401 with three questions, to be answered with a score from a scale from 1 to 5 where 1 represent the worst score and 5 represents the best score:

- How easy was it to reach your goal?
- Was the route clean and beautiful?
- Did you feel connected to other people?

In the example of Fig. 4 these questions were determined based on factor analysis of a survey conducted on a plurality of runners. It is to be appreciated that other questions and/or scoring systems could be used in other examples of the disclosure.

In this example the user 401 can provide the feedback by answering three simple questions. This may be convenient for the user 401 and avoids the user 401 having to input large amounts of data. For example, the user 401 does not need to individually assess each of the values assigned to the parameters for each of the segments on the route. Also the questions are easy for a user 401 to understand and do not require the user 401 to have knowledge of the various segments, parameters and how they are used to determine the route.

In some examples the feedback questions may be provided to the user once it has been determined that the route has been completed by the user 401. For instance the position of the apparatus and or user 401 could be monitored and once it has been determined that the user 401 has completed the route the feedback questions could be provided to the user 401.

Once the user 401 has answered the three questions then the information obtained in these answers can be provided as an input 433 and used to update the values assigned to the parameters for the segments and/or the weighting factors assigned to the parameters or sub-components of the parameters.

In some examples the values of the parameters may be unchanged but the values of one or more weighting factors could be adjusted. For example, if the user feedback was negative on the beauty and cleanliness of the path, the relative weighting of the scenery dimension is increased. In some examples the weighting factors could be created in response to the user feedback 401. This may provide for a personalised route planning system.

As the user 401, or a plurality of users, complete a plurality of different routes a learning set can be built which can be used to determine new parameter values and/or weighting factors. In some examples machine learning, or any other suitable algorithms can be used to determine the new parameter values and/or weighting factors.

In the example shown in Fig. 4 a measured parameter value is be assigned to a respective segment independently of the selected category of activities. The measured values are then adjusted by one or more weighting factors where the weights of the weighting factors are dependent on the selected category of activity. The weights of the weighting factors may therefore provide an association between an expected physiological effect on the user traversing a respective one of the plurality of segments and the intended physiological outcome defined by the selected category of activity.

In other examples the parameter values that are assigned to each of the segments could be different for each selected category of activity. For instance different methods of obtaining a measurement could be used for different selected categories of activity and this could be used to provide different measured values for the respective categories of activity.

In the example shown in Fig. 4 the values of the parameters assigned to the segments are obtained by accessing one or more information sources 411. In some examples the parameters and their values could be pre-stored in the memory 107 of the apparatus 101. In such examples this can enable the apparatus 101 to perform the described methods and provide a determined route without having to connect to another network such as the internet.

Examples of the disclosure provide apparatus, methods and computer programs that enable routes to be determined for a user 401 performing an activity to help the user achieve a physiological outcome. In examples of the disclosure the user 401 can select between different available categories. This provides for a system that is simple and intuitive for the user to use.

In examples of the disclosure the available categories of activity can be provided based on the analysis of the habits and motivations of users. This enables the system to cater for users having different habits and motivations.

Examples of the disclosure also enable a user 401 to provide feedback in relation to the route that has been provided for them. This feedback can then be used to update the system and apparatus 101 so as to improve the performance of the apparatus 101 and system. This may provide for a dynamic system which can be updated as it is used by a user 401.

In some examples the feedback can be given as a response to one or more questions. Providing a small number of questions to enable feedback to be obtained may provide for a convenient method of obtaining the feedback from the users 401.

Dividing the routes into segments and assigning values for a plurality of different parameters for the segments enables a variety of different parameters to be taken into account. This can improve the accuracy with which a route can be determined to enable an intended physiological outcome to be achieved. It may also enable different parameters to be used for different categories or for different types of activity or in different implementations of the disclosure.

The systems, apparatus, methods and computer programs may use machine learning which can include statistical learning. Machine learning is a field of computer science that gives computers the ability to learn without being explicitly programmed. The computer learns from experience E with respect to some class of tasks T and performance measure P if its performance at tasks in T, as measured by P, improves with experience E. The computer can often learn from prior training data to make predictions on future data. Machine learning includes wholly or partially supervised learning and wholly or partially unsupervised learning. It may enable discrete outputs (for example classification, clustering) and continuous outputs (for example regression). Machine learning may for example be implemented using different approaches such as cost function minimization, artificial neural networks, support vector machines and Bayesian networks for example. Cost function minimization may, for example, be used in linear and polynomial regression and K-means clustering. Artificial neural networks, for example with one or more hidden layers, model complex relationship between input vectors and output vectors. Support vector machines may be used for supervised learning. A Bayesian network is a directed acyclic graph that represents the conditional independence of a number of random variables.

The above described examples find application as enabling components of:
automotive systems; telecommunication systems; electronic systems including consumer electronic products; distributed computing systems; media systems for generating or rendering media content including audio, visual and audio visual content and mixed, mediated, virtual and/or augmented reality; personal systems including personal health systems or personal fitness systems; navigation systems; user interfaces also known as human machine interfaces; networks including cellular, non-cellular, and optical networks; ad-hoc networks; the internet; the internet of things; virtualized networks; and related software and services.

The term 'comprise' is used in this document with an inclusive not an exclusive meaning. That is any reference to X comprising Y indicates that X may comprise only one Y or may comprise more than one Y. If it is intended to use 'comprise' with an exclusive meaning then it will be made clear in the context by referring to 'comprising only one...' or by using 'consisting'.

In this description, reference has been made to various examples. The description of features or functions in relation to an example indicates that those features or functions are present in that example. The use of the term 'example' or 'for example' or 'can' or 'may' in the text denotes, whether explicitly stated or not, that such features or functions are present in at least the described example, whether described as an example or not, and that they can be, but are not necessarily, present in some of or all other examples. Thus 'example', 'for example', 'can' or 'may' refers to a particular instance in a class of examples. A property of the instance can be a property of only that instance or a property of the class or a property of a subclass of the class that includes some but not all of the instances in the class. It is therefore implicitly disclosed that a feature described with reference to one example but not with reference to another example, can where possible be used in that other example as part of a working combination but does not necessarily have to be used in that other example.

Although embodiments have been described in the preceding paragraphs with reference to various examples, it should be appreciated that modifications to the examples given can be made without departing from the scope of the claims.

Features described in the preceding description may be used in combinations other than the combinations explicitly described above.

Although functions have been described with reference to certain features, those functions may be performable by other features whether described or not.

Although features have been described with reference to certain embodiments, those features may also be present in other embodiments whether described or not.

The term 'a' or 'the' is used in this document with an inclusive not an exclusive meaning. That is any reference to X comprising a/the Y indicates that X may comprise only one Y or may comprise more than one Y unless the context clearly indicates the contrary. If it is intended to use 'a' or 'the' with an exclusive meaning then it will be made clear in the context. In some circumstances the use of 'at least one' or 'one or more' may be used to emphasis an inclusive meaning but the absence of these terms should not be taken to infer and exclusive meaning.

The presence of a feature (or combination of features) in a claim is a reference to that feature or (combination of features) itself and also to features that achieve substantially the same technical effect (equivalent features). The equivalent features include, for example, features that are variants and achieve substantially the same result in substantially the same way. The equivalent features include, for example, features that perform substantially the same function, in substantially the same way to achieve substantially the same result.

In this description, reference has been made to various examples using adjectives or adjectival phrases to describe characteristics of the examples. Such a description of a characteristic in relation to an example indicates that the characteristic is present in some examples exactly as described and is present in other examples substantially as described.

Whilst endeavoring in the foregoing specification to draw attention to those features believed to be of importance it should be understood that the Applicant may seek protection via the claims in respect of any patentable feature or combination of features hereinbefore referred to and/or shown in the drawings whether or not emphasis has been placed thereon.

**Claims**

1. An apparatus comprising means for:

    detecting an input that causes selection of a category of activity wherein the category of activity defines an intended physiological outcome for a user performing the activity; and
    determining a route for the selected category of activity;
    wherein the route comprises a plurality of segments and values of one or more parameters are assigned to one or more of the plurality of segments where the values of the parameters provide a respective level of association of the one or more parameters with the intended physiological outcome defined by the selected category of activity; and
    wherein determining a route for the selected category of activity comprises selecting segments from the plurality of segments based on the values of the one or more parameters.

2. An apparatus as claimed in any preceding claim wherein weights are assigned to respective ones of the values, where the weights are dependent upon the selected category of activity and where respective weights provide an association between an expected physiological effect on the user traversing a respective one of the plurality of segments and the intended physiological outcome defined by the selected category of activity; and
wherein determining a route for the selected category of activity comprises selecting segments from the plurality of segments as a function of the weighted values of the one or more parameters.

3. An apparatus as claimed in any preceding claim wherein the physiological outcome comprises a mental condition of the user.

4. An apparatus as claimed in any preceding claim wherein selecting a category of activity comprises selecting one of a predefined plurality of categories.

5. An apparatus as claimed in claim 4 wherein the predefined categories comprise any one or more of; mindfulness, vibrancy.

6. An apparatus as claimed in any of claims 2 to 5, wherein the different parameters have different weights for the different categories of activities.

7. An apparatus as claimed in any preceding claim wherein the parameters comprise parameters which affect the user's physical or mental state as they are traversing the route.

8. An apparatus as claimed in any preceding claim wherein the parameters comprise any one or more of; smell, sound, scenery, ground, obstacles, traffic volume, safety.

9. An apparatus as claimed in any preceding claim wherein determining a route for the selected category of activity comprises selecting segments from the plurality of segments so as to provide a cumulative value of the one or more parameters for the selected category activity above a threshold level.

10. An apparatus as claimed in any preceding claim wherein determining a route for the selected category of activity comprises selecting segments from the plurality of segments so as to optimise the cumulative values of the one or more parameters.

11. An apparatus as claimed in any of claims 9 to 10 wherein the cumulative value comprises a weighted value.

12. An apparatus as claimed in any preceding claim wherein the means are configured to detect one or more inputs indicating whether or not a user considers the route to satisfy the selected category of activity.

13. An apparatus as claimed in claim 12 wherein the one or more inputs indicating whether or not a user considers the route to satisfy the selected category of activity are used to update the values of the parameters assigned to the plurality of segments.

14. A method comprising:

detecting an input that causes selection of a category of activity wherein the category of activity defines an intended physiological outcome for a user performing the activity; and
determining a route for the selected category of activity;
wherein the route comprises a plurality of segments and values of one or more parameters are assigned to one or more of the plurality of segments where the values of the parameters provide a respective level of association of the one or more parameters with the intended physiological outcome defined by the selected category of activity; and
wherein determining a route for the selected category of activity comprises selecting segments from the plurality of segments based on the values of the one or more parameters.

15. A computer program comprising computer program instructions that, when executed by processing circuitry, cause:

detecting an input that causes selection of a category of activity wherein the category of activity defines an intended physiological outcome for a user performing the activity; and

determining a route for the selected category of activity;

wherein the route comprises a plurality of segments and values of one or more parameters are assigned to one or more of the plurality of segments where the values of the parameters provide a respective level of association of the one or more parameters with the intended physiological outcome defined by the selected category of activity; and

wherein determining a route for the selected category of activity comprises selecting segments from the plurality of segments based on the values of the one or more parameters.

*101*

113

Controller — 103

Processor — 105

Memory — 107

Computer program — 109

Computer program code — 111

# FIG. 1

201

101

Controller ~103

203~ User interface ←→ Processor ~105

Memory ~107

Computer program ~109

Computer program code ~111

FIG. 2

Detect selection of category of activity ~301

Determine route for selected category of activity ~303

FIG. 3

*411*    *411*    *411*

*405*

Social media data    OSM data    Other open data

*403*

Category route length

*401*

Category

Segment values    *433*

Running length

Geographical routing system

Recommened route

Recommened route    *423*

*421*

*407*

Performance
○ ○ ○ ○ ○

Environment
○ ○ ○ ○ ○

Social connectedness
○ ○ ○ ○ ○

Post-run questionnaire    *409*

*FIG. 4*

Europäisches
Patentamt

European
Patent Office

Office européen
des brevets

# EUROPEAN SEARCH REPORT

Application Number

EP 19 15 6190

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2014/303892 A1 (MORLOCK CLAYTON RICHARD [US]) 9 October 2014 (2014-10-09) * abstract; figure 5 * * paragraphs [0001], [0007] - [0025] * * paragraphs [0054] - [0063] * * paragraph [0130] * * paragraphs [0201] - [0240] * | 1-15 | INV. G06Q10/04 G01C21/34 |
| X | US 2011/137551 A1 (PERI OMER SHMUEL [IL]) 9 June 2011 (2011-06-09) * abstract; figures 1-5 * * paragraphs [0021] - [0049] * | 1-15 | |
| A | US 2008/293430 A1 (BLOM JAN [FI] ET AL) 27 November 2008 (2008-11-27) * abstract; figures 3-5 * * paragraph [0007] * * paragraphs [0036] - [0055] * | 1-15 | |
| | | | TECHNICAL FIELDS SEARCHED (IPC) |
| | | | G06Q G01C |

The present search report has been drawn up for all claims

| Place of search | Date of completion of the search | Examiner |
|---|---|---|
| Munich | 8 April 2019 | Laurentowski, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 19 15 6190

This annex lists the patent family members relating to the patent documents cited in the above-mentioned European search report.
The members are as contained in the European Patent Office EDP file on
The European Patent Office is in no way liable for these particulars which are merely given for the purpose of information.

08-04-2019

| Patent document cited in search report | | Publication date | Patent family member(s) | | Publication date |
|---|---|---|---|---|---|
| US 2014303892 | A1 | 09-10-2014 | CN | 104081158 A | 01-10-2014 |
| | | | EP | 2780667 A2 | 24-09-2014 |
| | | | JP | 6199875 B2 | 20-09-2017 |
| | | | JP | 2015504557 A | 12-02-2015 |
| | | | KR | 20140091606 A | 21-07-2014 |
| | | | US | 2014303892 A1 | 09-10-2014 |
| | | | US | 2017082443 A1 | 23-03-2017 |
| | | | WO | 2013075072 A2 | 23-05-2013 |
| US 2011137551 | A1 | 09-06-2011 | NONE | | |
| US 2008293430 | A1 | 27-11-2008 | CN | 101680767 A | 24-03-2010 |
| | | | EP | 2149032 A1 | 03-02-2010 |
| | | | JP | 5027920 B2 | 19-09-2012 |
| | | | JP | 2010531432 A | 24-09-2010 |
| | | | KR | 20100013332 A | 09-02-2010 |
| | | | TW | 200909782 A | 01-03-2009 |
| | | | US | 2008293430 A1 | 27-11-2008 |
| | | | WO | 2008142605 A1 | 27-11-2008 |
| | | | ZA | 200908914 B | 25-05-2011 |

EPO FORM P0459

For more details about this annex : see Official Journal of the European Patent Office, No. 12/82